# EUROPEAN PATENT APPLICATION

(11) **EP 3 241 820 A1**
(43) Date of publication of application: **08.11.2017**
(21) Application number: 16167958.4
(22) Date of filing: 02.05.2016
(51) Int. Cl.: C07C 51/47, C07C 51/493, C07C 53/08, C07C 55/10, C07C 59/08, C12P 7/40

(54) **RECOVERY OF CARBOXYLATES FROM FERMENTATION AND PRODUCTION OF ESTERS USING CO2 EXPANDED ALCOHOLS**

(71) Applicant: Technische Universiteit Delft, 2628 CN Delft (NL); Stichting voor de Technische Wetenschappen, 3527 JP Utrecht (NL)
(72) Inventor: STRAATHOF, Adrianus Johannes Jozef, 2600 AA Delft (NL); CABRERA RODRIGUEZ, Carlos Ivan, 2600 AA Delft (NL)
(74) Representative: Vogels, Leonard Johan Paul

(57) **Abstract**

The present invention is in the field of a method for recovering a carboxylate, using an anion exchange resin capturing the carboxylate, from an aqueous solution comprising said carboxylate, batch wise or continuously, wherein use of raw material is reduced and if possible re-used. The present invention is in the field of green technology.

## Description

### FIELD OF THE INVENTION

The present invention is in the field of a method for recovering a carboxylate, from an aqueous solution comprising carboxylate, using an anion exchange resin capturing the carboxylate, batch wise or continuously, wherein use of raw material is limited and if possible re-used. The present invention is in the field of green technology.

### BACKGROUND OF THE INVENTION

Many compounds, such as those originating from natural sources such as sugar and cellulose, may be used as a starting point for forming further molecules. These compounds may be considered as chemical building blocks. Such compounds are a potential "carbon-neutral" feedstock for fuels and chemicals. An example is hydroxymethylfurfural (HMF), or 5-(Hydroxymethyl)furfural. Recently, an enzyme from *Cupriavidus basilensis* (HMF14) was used to convert HMF to FDCA using molecular oxygen. Also *Pseudomonas putida* can convert HMF to FDCA. Such bioconversion is typically effected in water, at ambient temperature and pressure.

HMF is typically produced from sugars, such as through dehydration of fructose. HMF is also naturally generated in sugar-containing food during heat-treatments and it is formed in the Maillard reaction. HMF can be converted to 2,5-dimethylfuran (DMF), which is a liquid biofuel. HMF can be converted into 2,5-furandicarboxylic acid (FDCA) by oxidation. It is noted that dehydration processes using hydroxymethylfurfural (HMF) as intermediate are generally non-selective, not cost effective and there is no industrially viable oxidation technology that can operate in concert with the necessary dehydration processes.

Several dicarboxylic acids are of industrial relevance, and so are their esters. FDCA and its esters are valuable sources in production of polyesters. FDCA can be used as an important renewable resource e.g. to substitute terephthalic acid (PTA) and to form polymers. There are various further derivatives of FDCA. FDCA undergoes reactions typical for carboxylic acids. FDCA has also been applied in pharmacology.
Other dicarboxylic acids are considered important platform chemicals such as fumaric, malic, itaconic and succinic acid. Due to their chemical functionality, succinic acid can be further transformed into different chemical derivatives and into polymers. Succinic acid undergoes reactions typical for carboxylic acids. The uses of succinic acid are broad in the chemical industry. Many microorganisms are able to convert carbohydrates to succinic acid. Recently, engineered *Corynebacterium glutamicum* strains, for example, have been used to produce succinic acid in high yields and concentrations.

Often carboxylic acids need to be obtained from solutions comprising aqueous carboxylates. For example, the most efficient bacterial fermentation methods for producing carboxylic acids may require a titration with a base to maintain a solution at a neutral pH, because the pKₐ values of the carboxylic acids produced are typically 3-5. The result thereof is a carboxylate solution with a pH above the pKₐ of the carboxylic acid. The industrial production of carboxylic acids from renewable resources via fermentation has however been hampered by an inefficient downstream processing (purification and further transformation). A problem for the recovery is that at the pH of the fermentation the acid is present in the dissociated form. Traditional recovery of carboxylic acids from this carboxylate solution coproduces high amounts of waste and/or consumes high amounts of energy. A reason considered is that at this pH the acid is in the dissociated form, and the primary recovery is limited to electrostatic interactions (e.g. anion/cation exchange, electrodialysis), precipitation or protonation of the carboxylate anion. Precipitation and protonation of the acids traditionally involves the formation of stoichiometric amount of salts as waste, while electrodialysis consumes high amounts of energy. This issue has been treated and solved separately for particular processing cases and they are discussed in the next section.

The above problems may be better understood as relating to three main issues. Firstly, most bacterial acid fermentations are carried out at a pH above the pKₐ of the carboxylic acid and hence a carboxylic acid is typically present as a carboxylate; the carboxylate counter-ion is then removed by acidification. This is a very polluting process, producing stoichiometric amounts of salts as by-products with little market value. Secondly, the purification of the obtained carboxylic acid (particularly those being highly water soluble) is a very intensive process in terms of materials, energy and unit operations. Finally, if carboxylate ester derivatives are wanted, the acid is typically purified first as regularly and thereafter esterified. This esterification reaction has inherent difficulties regarding chemical equilibrium. The above problems have been treated and solved separately only for particular processing cases. The main reason for the first mentioned problem is that most of the natural acid overproducer strains are bacteria and, in general, are only active at neutral pH. A partial solution for this is to produce a salt suitable of further thermal cracking, leading to an energy demanding process. The second mentioned problem can been overcome by lowering the solubility of the carboxylate salts by means of using a less soluble metal counter-ion (e.g. calcium or magnesium) and thus reducing the amount of required unit operations. Such an approach works at the expense of chemical consumption. Other alternatives capture the un-dissociated acid by means of hydrophobic interaction or hydrogen bonding using adsorption or extraction techniques. The desorption and back-extraction issue associated with those processes is sorted out by the use of chemicals and/or energy. For the last problem, regular strategies to overcome equilibrium limitations (i.e. removing products from reaction) have been used. Partial integrated schemes have certainly demonstrated some process advantages. No economically viable method has been provided however for the recovery of the carboxylic acids c.q. carboxylates.

Anion exchange resins may be used to recover carboxylates because of the high affinity of e.g. a quaternary ammonium group for the dissociated form of the carboxylic acid. In a prior art anion exchange process, a main drawback is the desorption of the carboxylate from the anion exchange resin. This desorption process involves adding an extra chemical (e.g. NaCl, H₂SO₄) which produces a stoichiometric amount of salt waste. Another option is to use methanol and H₂SO₄ to protonate the carboxylate anion, and then the carboxylic acid is esterified using the H2SO4 as catalyst. Unfortunately, in this case there is a stoichiometric amount of salt produced as waste.

Some recent publications relate to esterification of carboxylates. Examples thereof are López-Garzón et al. in "Direct downstream catalysis: From succinate to its diethyl ester without intermediate acidification", Chem. Engineering Journal 2012, Nr. 637 pp. 200-202, and López-Garzón et al. "Green upgrading of succinate using dimethyl carbonate for a better integration with fermentative production". Chem. Engineering Journal 2014, Nr. 52, p. 235.

Cainelli G. et al. in "The Use of Anion-Exchange Resins in the Synthesis of Esters from Carboxylic Acids and Alkylating Agents", Synthesis, pp. 723-724, 1975, consider the use of anion exchange resins for alkylation of carboxylate ions. The anions (substrates) tested were alkyl and aryl monocarboxylates. As alkylating agents, strong alkyl and aryl halides (mostly bromides) were used. In this process, the chemicals used are not considered safe, and the method is applied to chemically obtained carboxylates.

A process in which a carboxylic acid (namely lactic and succinic acids) or a carboxylate is extracted by an amine-based extractant (A) or a basic sorbent (B and C) is disclosed e.g. in US 5,132,456 B, US 5,412,126 B, US 5,965,771 B and US 6,316,668 B. The regeneration of an auxiliary phase is performed using ammonia or a low molecular weight amine (trimethylamine) which forms an amine-carboxylate salt that can be decomposed thermally yielding the desired carboxylic acid. The extraction/sorption stage is greatly affected by the solution pH and the basicity of the ion exchanger. In the case of succinic acid, at neutral pH, a mixture of acid and monovalent salt could be captured. The suggested regeneration could produce again such mixture since protons (H⁺) were added to the system. The process was subject to subsequent improvements in order to enhance the stability of the amine-carboxylate salt (C) and to reduce the amount of sorbed salt by means of carbon dioxide acidification (D). However, the complicated scheme detailed in the above mentioned patents does not solve the problematic succinic acid recovery at neutral pH and will produce, ultimately, a mixture of salts. In addition no ester derivatives are produced.

A process in which pure succinic acid is obtained from an ammonium succinate salt is mentioned in EP 1 005 562 B1. During fermentation wherein succinic acid is produced, the pH is controlled using ammonia and therefore yielding diammonium succinate. The salt is thereafter acidified using ammonium hydrogen sulphate producing succinic acid and ammonium sulphate. Succinic acid is then purified by dissolution in methanol and further crystallization. The produced ammonium sulphate is cracked at high temperature (300 °C), producing the required ammonium hydrogen sulphate and ammonia that are recycled to the acidification unit and fermenter, respectively. The main drawback of the process is considered to be the ammonium sulphate thermal cracking, which is energy demanding, produces corrosive fumes, and has mass and heat transfer problems.

In a paper by Lin S.K.C. et al., "Novel resin-based vacuum distillation-crystallisation method for recovery of succinic acid crystals from fermentation broths." Green Chemistry, 12, pp. 666-671, 2010 a method is mentioned in which succinic acid crystals are obtained from a neutral fermentation broth after treatment using a cation exchange resin. In the publication the concepts of acidification and salt-to-acid conversion are considered misleading and it is further suggested that the ionexchange based process does not require acidification. In fact, however, in the process acidification takes place when sodium cation from the carboxylate salt is exchanged by hydrogen ions (H⁺) from the resin. The publication claims a succinic acid recovery process from neutral fermentation broth without "direct" acidification, such definition is however inaccurate.

In a paper by Orjuela A., et al. "A novel process for recovery of fermentation-derived succinic acid." in Separation and Purification Technology, 83, 31-37, 2011, and the similar patent application WO 2011/069008 (A1) a method is mentioned for producing alkyl esters of carboxylates via acidification and esterification in alcohols. The process is considered particularly suitable for the production of diethyl succinate from succinate salts and ethanol using sulfuric acid for acidification. The process intends to avoid the acid purification prior to the esterification step. The advantages are limited to that aspect and to the purity of the obtained ester. Mineral acid is consumed and waste salts are produced. No integration with fermentation is claimed.

In a paper by Barve P., et al. "Preparation of pure methyl esters from corresponding alkali metal salts of carboxylic acids using carbon dioxide and methanol", in Industrial and Engineering Chemistry Research, 51, 1498-1505, 2012 and the similar patent application WO 2011/027211 (A3) production of alkyl esters from several solid metal carboxylates, acetates, lactates, salicylates and benzoates is mentioned. The process is intended to reduce or eliminate carboxylate acidification prior to esterification. For the production of esters, carbonic acid is used as acidifying agent and acid catalyst for the esterification with methanol. Carbonic acid is formed in-situ under the presence of low concentrations of water. Thereto, a carboxylate-rich fermentation stream should be firstly evaporated and the remaining solids be subjected to reaction. The reaction conditions are harsh (P = 20-60 bar and T = 175 °C). The reaction yields are around 80% under an excess of methanol. As a by-product metal carbonates are produced and their recycle to fermentation is suggested. For this process a low selectivity is expected. Also, a high energy consumption is expected with the evaporation of water to dry the metal salt of carboxylic acid prior to the esterification.

In a further patent application WO 2012/013793 (A1)a process is mentioned that involves the production of a carboxylate (lactate) by fermentation at near-to-neutral pH, by acidification of the carboxylate stream with a mineral acid and by purification of the carboxylic acid by cation-exchange chromatography. The waste effluent from the chromagraphic process (salts) is split by bipolar electrodialysis and the produced base and acid are recycled to the fermenter and acidification unit, respectively. The process is able to produce carboxylic acids from neutral fermentation broth at expenses of high energy consumption in the electrodialysis operation. The upstream and downstream sections are well integrated. Salt waste is produced after the chromatographic step. An ester derivative is not produced.

US 2011/0,244,534 A1 recites a process in which a calcium or magnesium salt of succinate is produced, separated and converted to a monovalent salt. The monovalent salt is further processed by conventional means. As a result, some savings in chemicals consumption are achieved.

In the MixAlco process, as described in Pham, V. et al. "Techno-economic analysis of biomass to fuel conversion via the MixAlco process." in J. Ind. Microbial Biotechnol. 2010, nr. 37, p. 1157 and WO 2008/070561 A1, a biodegradable material is converted into mixed alcohol fuels containing predominantly 2-propanol, but also higher alcohols up to 7-tridecanol. The feedstock is treated with lime to increase its digestibility. Then, it is fed to a fermentor in which a mixed culture of acid-forming microorganisms produces carboxylic acids. Calcium carbonate is added to the fermenter to neutralize the acids to their corresponding carboxylate salt. The dilute (approximately 3%) carboxylate salts are concentrated to 19% using an amine solvent that selectively extracts water. Drying is completed using multi-effect evaporators. Finally, the dry salts are thermally converted to ketones which subsequently are hydrogenated to alcohols. The main limitation of the process is the high energy consumption for drying the amine salts with the water evaporators.

The present invention therefore relates to a method for recovering carboxylates and carboxylic acids, which overcomes one or more of the above disadvantages, such as minimizing chemical consumption, using renewable material, providing a highly pure compound, being non-toxic, at low (energy) costs, without jeopardizing functionality and advantages.

### SUMMARY OF THE INVENTION

The present invention relates in a first aspect to a method for recovering carboxylates and carboxylic acids, according to claim 1. Therein a CO₂ expanded alcohol and a resin are used. The term "carboxylate" may relate to an acid form thereof, depending on a pH of the solution, and vice versa; depending on the pH the acid will have all its protons, or one or more protons, as applicable, have been released to the solution. The terminology "CO₂ expanded alcohol" relates to an alcohol in which CO₂ is dissolved and as a result thereof the volume of the alcohol increases (expands). An amount of CO₂ dissolved varies typically from 0.01-0.5 mole CO₂/mole alcohol, preferably 0.02-0.3 mole CO₂/mole alcohol, more preferably 0.05-0.2 mole CO₂/mole alcohol, such as 0.07-0.15 mole CO₂/mole alcohol, depending on e.g. type of alcohol, pressure, and temperature.

The invention solves the above described problems by integrating e.g. bio-based production of a target carboxylate, its capture and chemical upgrading. The present method is equally well suited for carboxylates produced in other ways. The present method is designed to produce and ester derivative from carboxylic acids e.g. produced by fermentation without a previous acidification of an aqueous solution, such as the broth. The synthesis of such a derivative provides a higher level of process integration thereby minimizing chemical consumption, waste generation, use of energy, and unit operations. Therein consumption of chemicals such as inorganic acids and bases, and associated production of inorganic waste salt are prevented, contrary to conventional methods that produce up to 1 kg salt per kg ester. These advantages improve the sustainability of the present method.

The present method relate to recovery and esterification of carboxylates using CO₂-expanded alcohols. Using this method, the carboxylates are recovered with an anion exchange resin, and the CO₂-expanded alcohol is used for the desorption and further esterification of the carboxylates. Recovery of a carboxylic acid from the CO₂-expanded alcohol without performing esterification is also possible. The main advantages are: non stoichiometric waste production, low pressures of CO₂ needed and integration of the unit operations. For example, using CO₂ expanded methanol, inventors achieved a yield of 0.79 mol acetic acid/acetateᵢₙ (i.e. 79%) in the desorption step at 10 bar of CO₂, and complete conversion (1.03 mol methyl acetate/acetateᵢₙ) in the combined desorption-esterification at 5 bar of CO₂ and 60 °C. The method is an efficient alternative for the recovery of different carboxylates produced by fermentation, typically at pH > pKa, such as lactate (0.70 methyl lactate/lactateᵢₙ) and succinate (0.18 dimethyl succinate/succinateᵢₙ). Moreover, the method can be applied with other alcohols and likewise a mixture of alcohols. A yield of 0.67 mol of ethyl acetate/mol acetateᵢₙ was obtained with CO₂-expanded ethanol. The method is versatile and can be adapted for several applications. Different process operations, temperature and reactor setup might be used depending on the specific application.

The present method may include four main operations:
1) A carboxylate production, such as a bio-based production (fermentative or enzymatic) which is carried out while controlling the pH at neutral values, such as by using an inorganic base, e.g. NaOH, CaO, bicarbonate and carbonate.
2) The carboxylate is captured using an anion exchange sorption step which may liberate the inorganic base required for step 1. In particular cases this is bicarbonate or carbonate.
3) The sorbed carboxylate is desorbed and protonated using a CO₂ expanded alcohol. Examples of such alcohols are C₁-C₆ straight or branched, and/or optionally being cyclic, primary or secondary alcohols, such as methanol, ethanol, propanol, isopropanol, butanol, heptanol, hexanol, benzyl alcohol, etc. In this step, the resin used is regenerated simultaneously to the bicarbonate form.
4) The carboxylic acid is dissolved in the alcohol now, and esterification may be performed using an acid homogeneous or heterogeneous catalyst.

The above method is considered an improvement of prior art carboxylate downstream processes and organic synthesis techniques. It is considered to be built on the current state of the art.

It is noted that CO₂ per se has been used to convert a carboxylate into its equivalent carboxylic acid in the prior art, albeit this has been done in combination with an aqueous phase. To the knowledge of the inventors CO₂ expanded alcohols have not been used for the desorption of carboxylates from an anion exchange resin and a further esterification at mild conditions. It is found that a solubility of CO₂ in e.g. methanol and ethanol is 10-20 times higher than in with water (at similar conditions), which provides certain advantages, such as an operation at milder conditions.

The above mentioned integration of the desorption-reaction and the regeneration of the strong anion exchange resin is considered novel. Furthermore, the way of supplying the required inorganic base in some of the exemplary fermentations by integrating both fermentation and sorption processes is also considered novel.

Examples of suitable carboxylic acids (and carboxylates thereof) are C₂-C₂₀ carboxylic acids, optionally comprising at least one further carboxylic group, such as 1-2 further carboxylic groups, and/or a further comprising at least one alcohol group, such as 1-2 alcohol groups, and/or optionally being cyclic, such as comprising a cyclohexane or cyclopentane group, and/or comprising an aromatic moiety, such as a benzene group, such as formic acid, acetic acid, propionic acid, butyric acid, valeric acid, caproic acid, enanthic acid, caprylic acid, pelargonic acid, capric acid, undecanoic acid, lauric acid, tridecyclic acid, myristic acid, pentadecanoic acid, palmitic acid, margaric acid, stearic acid, nonadecyclic acid, arachidic acid, lactic acid, succinic acid, levulinic acid, itaconic acid, 2,5-furandicarboxylic acid (FDCA), phthalic acid, acrylic acid, adipic acid, benzoic acid, fumaric acid, 3-hydroxy butyric acid, malic acid, pimelic acid, 3-hydroxyproprionic acid, sugar acids such as uronic acids and aldonic acids, vanillic acid, ferulic acid, and alginic acid.

The present method can start with a fermentative or enzymatic of a carboxylate. Such is typically carried out using an aqueous solution while controlling the pH at neutral values (pH 4-10) using e.g. an inorganic base, which values are typically above the pKₐ of the carboxylate. The present method can also start with a chemical production of an aqueous carboxylate at above the pKₐ. After the production step, the aqueous carboxylate is captured using an anion exchange sorption step which may liberate the inorganic base above.

Thereby the present invention provides a solution to one or more of the above mentioned problems.

Advantages of the present description are detailed throughout the description.

### DETAILED DESCRIPTION OF THE INVENTION

The present invention relates in a first aspect to a method of recovery of carboxylic acids comprising the steps of providing the carboxylic acid in an aqueous solution, performing an anion exchange sorption step using a resin capturing the carboxylate, desorbing the carboxylate and protonating the carboxylate with a CO₂ expanded alcohol and simultaneously regenerating the resin (to a bicarbonate form).

The present results and considerations are submitted for publication in an article by Cabrera-Rodriguez et al., entitled "Recovery and esterification of carboxylates produced by fermentation at pH > pKa using CO2-expanded alcohols", which publication and its contents are hereby incorporated by reference.

In an example of the present method relating to methanol as expanded alcohol and acetate as carboxylate, the equilibrium reactions considered relevant are: binding the acetate to a resin (denoted Q⁺); protonation of the acetate bound to the resin via formation of a methyl carbonate anion (from MeOH and CO₂, R.1); protonation and release of the acetate under simultaneous formation of a bound bicarbonate anion (R.2); hydrolysis of a bound methyl carbonate anion under simultaneous release of methanol(R.3); formation of dimethyl carbonate and bound hydroxide from the bound methyl carbonate anion and methanol (R.4); and conversion of bound hydroxide with carbon dioxide to the bicarbonate form of the resin (R.5). 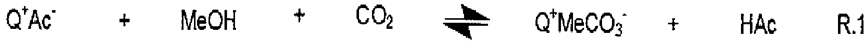 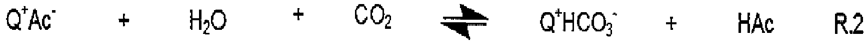 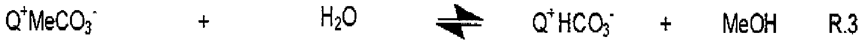 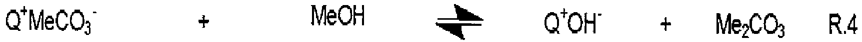 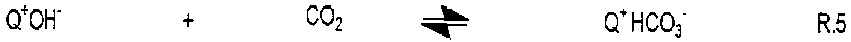 As a result, the acetic acid (protonated acid) formed is in solution with methanol and CO₂. This solution can be used to produce an ester (R.6), in which case, the excess of methanol and CO₂ direct the equilibrium to the products (ester and water) formation.

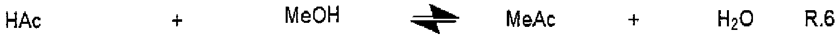

A further method step might be a distillation e.g. of the protonated acid, an esterification e.g. of the acid, or any other reaction step.

In the present method the carboxylate is soluble in an alcohol of the method and in water, as e.g. it is produced in an aqueous solution, such as a broth. In an aspect the water soluble carboxylate allows for a high degree of integration of process steps.

In an example of the present method the carboxylate is produced by biotransformation, such as microbial or enzymatic transformation, or a combination thereof, such as a fermentation process, from a microbial process, from hydrolysis of biomass, from a chemical conversion, or from waste water, such as by conversion by a microorganism from a broth, such as with C. *glutamicum or P. putida,* preferably conversion in an aqueous medium, wherein a pH of the medium is from 4-10, preferably from 5-9, more preferably from 6-8. Therewith high yields are obtained. Further a high degree of integration with such transformation may be obtained.

In an example of the present method the the aqueous solution comprises a broth.

Any combination of carboxylates may be used. Such indicates the present method is relatively robust and can be used over a broad scope of carboxylates and mixtures thereof. Such is particularly relevant if the one or more carboxylates are produced by biotransformation.

In an example, methyl acetate, methyl acrylate, dimethyl adipate, dimethyl benzoate, methyl butyrate, dimethyl FDCA, dimethyl fumarate, methyl 3-hydroxybutyrate, methyl 3-hydroxypropionate, dimethyl itaconate, D- or L-methyl lactate, dimethyl malate, dimethyl pimelate, methyl propionate, dimethyl succinate, and combinations thereof are formed. Also using ethanol, ethyl acetate, ethyl acrylate, diethyl adipate, diethyl benzoate, ethyl butyrate, diethyl FDCA, diethyl fumarate, ethyl 3-hydroxybutyrate, ethyl 3-hydroxypropionate, diethyl itaconate, D- or L-ethyl lactate, diethyl malate, diethyl pimelate, ethyl propionate, diethyl succinate, and combinations thereof are formed. The above esters relate to readily available carboxylates, which can be formed relatively easy, providing the advantages of the invention.

In an example of the present method the ester is a mono-, di- or tri ester, preferably a diester in its final form. The ester preferably comprises one, two or three alkyl groups as indicated below, such as methyl, ethyl, propyl and combinations thereof.

In an example of the present method the water soluble carboxylate comprises one or more of Na⁺, NH4⁺, K⁺, Mg2⁺, Ca2⁺, and combinations thereof.

In an example of the present method the carboxylate is sorbed by an anion exchanger from an aqueous solution, i.e. fermentation broth with or without previous purification. In a general example, the carboxylate is sorbed in a fixed bed operation after protein and cell removal performed by conventional means such as filtration operations. In a particular example, the carboxylate is sorbed in expanded bed mode without the need of protein or cell removal. In a particular instance of such example, both processes, i.e. biotransformation and sorption, are integrated and occur simultaneously.

In an example of the present method the carboxylate is sorbed not (just) by an anion exchange resin but (also) by an ionic liquid with a quaternary ammonium, phosphonium or guanidium functional group.

In an example of the present method a pH of the aqueous solution is controlled by addition of a suitable amount of base selected from at least one of oxide, hydroxide, bicarbonate and carbonate, preferably bicarbonate and carbonate.

In an example of the present method the base is liberated from the resin during sorption of the carboxylate.

In an example of the present method the pH of the aqueous solution is larger than the pKₐ of the acid of the carboxylate, preferably pH>(pKₐ +0.5), more preferably pH>(pKₐ +1.0).

In an example of the present method the alcohol comprises at least one of a C₁-C₁₂ straight or branched, primary or secondary alcohol, such as methanol, ethanol, propanol, isopropanol, butanol, heptanol, 3-methyl-1-butanol, (trans)-3,7-Dimethyl-2,6-octadien-1-ol, and hexanol, and optionally one of water-miscible solvents selected from acetone, acetonitrile, and tetrahydrofuran.

In an example of the present method a CO₂ pressure is from 0,5-200 * 10⁵ Pa (0,5-200 bar), preferably 1-100 * 10⁵ Pa, more preferably 5-50 * 10⁵ Pa, such as 10-20 * 10⁵ Pa.

In an example of the present method the desorption is performed at a temperature of 0-100 °C, preferably 20-60 °C.

In an example of the present method the protonated carboxylate is dissolved in the CO₂ expanded alcohol.

In an example of the present method the protonated carboxylate is esterified under homogeneous acid or heterogeneous catalyst conditions.

In an example of the present method a catalytic agent is provided, selected from H₂SO₄, HCl, a cation exchange resin comprising sulfonic groups, enzymes, and zeolites.

In an example of the present method the resin is dried, such as by desiccation, by heating, or by washing using a suitable drying solvent, preferably an alcohol, such as ethanol or methanol.

In an example of the present method prior to the anion exchange sorption step water is removed from the resin, preferably removed to a concentration of less than 1% (w/w water/resin), more preferably less than 0.1%.

In an example of the present method the esterification is performed during a period of 0.1 -10 h, preferably 0.2-5 h, more preferably 0.5-2 h.

In an example of the present method the integration of the desorption and esterification is carried out in a simultaneous 1-compartment system, in a consecutive 2-compartment system, or in a simultaneous 2-compartment system.

In an example of the present method the carboxylic acids in the CO₂ expanded alcohol is recovered, such as by at least one of evaporation of the alcohol and CO₂, crystallization of the carboxylic acids, and anti-solvent addition.

In an example of the present method prior to the anion exchange sorption step water is removed from the resin, preferably removed to a concentration of less than 1% (w/w water/resin), more preferably less than 0.1%.

In an example of the present method water is removed, such as by reactive distillation, preferably to an amount of less than 5% (v/v, water/total volume), more preferably < 3%.

In an example of the present method the ester is formed at a temperature of 20-140 °C, preferably 25-120 °C, at a pressure of 80-900 kPa, such as at 90-500 kPa, preferably at or near ambient conditions. Such temperatures and pressures are relatively mild.

In an example of the present method the ester is purified without much trouble, such as by crystallisation, by distillation, or combination thereof.

In an example of the present method the carboxylate is purified.

The one or more of the above examples and embodiments may be combined, falling within the scope of the invention.

### EXAMPLES

The invention is further detailed by the accompanying examples and figures, which are exemplary and explanatory of nature and are not limiting the scope of the invention. To the person skilled in the art it may be clear that many variants, being obvious or not, may be conceivable falling within the scope of protection, defined by the present claims.

The aim of the examples is to explore the effect of protonating a carboxylate anion recovered by an anion exchange resin with a CO₂-expanded alcohol, and to further esterify the carboxylic acid. In this approach, the starting material is an anion exchange resin loaded with a carboxylate. Then, inventors use a CO₂/alcohol system for the desorption of the carboxylates from the anion exchange resin. These CO₂/alcohol systems at relatively low pressures (<P_{c} (CO₂)) are known as gas-expanded liquids. Inventors studied the effect of the CO₂ pressure on the desorption of acetate in methanol and ethanol; the effect of water on the protonation and esterification steps; and the application of the technology for other carboxylates produced by fermentation, e.g. lactate and succinate. It is observed that microbial or enzymatic production of carboxylic acids is usually more efficient at neutral pH than at low pH, as uncharged carboxylic acids may be toxic or inhibiting.

### Experimental

### Material and Methods

### Materials

Analytical grade chemicals were used as bought: potassium acetate (≥99.9%), Dowex Marathon MSA resin (type I; macroporous, in a chloride form, minimum exchange capacity 1.1 eq/L), methyl acetate (≥99.9%), dimethyl carbonate (≥99.9%), anhydrous methanol (≥99.8%), succinic acid (≥99%), dimethyl succinate (≥99%), monomethyl succinate (≥95%), lactic acid (≥90%), methyl lactate (≥97%), and anhydrous ethyl acetate (≥99.8%) (Sigma-Aldrich), acetic acid (≥99% from JT. Baker B.V.). Ethanol extra dry/absolute (≥99.5%) was purchased from Fischer Scientific. Carbon dioxide (≥99.8%) was purchased as compressed gas from the Linde Group. Amberlyst15 (Serva, Heildelberg) hydrogen form (4.7 meq/g by dry weight) was washed with MeOH and dried at 60 °C in an oven before use. Aqueous solutions were prepared by diluting the potassium acetate salt to the required concentrations (20 g/L) with deionized water from a Milli-Q water purification system (Millipore).

### Resin Preparation and adsorption

A column elution technique was used to convert the Dowex Marathon MSA (Sigma-Aldrich) resin from a chloride form thereof to an acetate form. The resin was washed with a constant flow (2 mL/min) of a potassium acetate solution (20 g/L), until the observed concentration of chloride in an outlet was lower than 9 mg/L and a UV-Vis absorbance at 220 nm at the outlet of the column is constant. The chloride concentration in the outlet samples were in addition measured with a colorimetric method (QuantiChrom™). The resin was washed three times in a batch with 50 mL deionized water and an excess water was removed using a Millipore Steriflip 60 µm nylon net filtration unit, at - 2000 Pa (-20 mbar). Subsequently, the resin was washed three times with 30 mL methanol and filtered with the same system. Finally, the resin was dried in an oven at 60 °C for 16 h. The elemental composition of the surface of the resin was measured with X-ray photoelectron spectroscopy (XPS), the water concentration in the resin in each step was measured by thermogravimetric analysis (TGA).

### Desorption with a carbon dioxide expanded alcohol

The desorption of acetate from the above resin was performed in a 50 mL Büchi glass stirred autoclave reactor. The reactor was equipped with a magnetically driven four blade impeller controlled by an overhead motor, a thermocouple for temperature control, a pressure sensor, a pressure relief valve, a nitrogen and carbon dioxide inlet, and ports for reagent addition and sampling.

The desorption experiments were performed by adding 0.5-1.0 g of Dowex MSA-acetate (2.2 mmol acetate/g dry resin) to 30 g of anhydrous methanol (or in an equivalent experiment ethanol). Thereafter the reactor was flushed with nitrogen to obtain an inert atmosphere. Agitation by the impeller was achieved at 600 rpm. In control experiments a pressure of 500 kPa (5 bar) of nitrogen was maintained during the experiment. For the other experiments the system was flushed five more times with carbon dioxide, and then the pressure was set to the desired value (200, 500 or 1000 kPa respectively; 2, 5 or 10 bar of CO₂). The reactor was then re-pressurized with carbon dioxide until the pressure measured was constant over time. The experiments were performed for 4 h and in duplicate. Liquid samples for analysis were obtained at the set pressure. Initial samples of the solvent, and final samples were analyzed for the specific methyl esters, carboxylic acid and water content, respectively.

### Esterification reactions

Subsequent esterification reactions were carried out in closed glass reaction tubes in a Greenhouse Plus Parallel Synthesizer (Radleys). The experiments were performed in duplicate. In a typical experiment 4 g of a 2% or 4% w/w (acid/alcohol) of acetic acid in MeOH was added to the reaction tubes. A catalyst was added to the reaction tubes (0.06 g of dried Amberlyst15 hydrogen form or 4 µL of H₂SO₄, respectively). The tubes were flushed with N₂ to obtain an inert atmosphere. The reaction conditions were kept at 60 °C and the reaction mixture was agitated with a magnetic stirrer at 600 rpm in a Greenhouse Plus Parallel Synthesizer for 4 h. Final samples were analyzed for methyl acetate, acetic acid and water content, respectively.

For simultaneous desorption and esterification reactions, a similar procedure as explained in the above section was followed. The difference was the addition of 0.5 g of prewashed Amberlyst15 in a separate chamber in the reactor to catalyze the esterification reaction. The reaction was performed at 60 °C or 78 °C for 4 h. Final samples were analyzed for the methyl esters, carboxylic acids and water content, respectively.

### Analytical Methods

Dimethyl carbonate, methyl acetate, ethyl acetate, dimethyl succinate, methyl lactate and acetic acid were analyzed by gas chromatography (GC) using a ZB-WAXplus column (20 m length x 0.18 mm internal diameter, 0.18 µm film) and a flame ionization detector (FID). Injection and detector conditions were maintained during analysis. Liquid samples were conditioned with formic acid and anisole as internal standard. The sample (0.5 µL) was injected at 200 °C with a split flow of 30 mL/min. The oven temperature was maintained at 60 °C for 10 min, then a 10 °C/min temperature ramp was used to increase the temperature to 200 °C. Succinic acid, methyl succinate and lactic acid concentrations were analyzed on a Waters HPLC system using a Bio-Rad Aminex HPX-87H column (78x300 mm) at 60 °C. Phosphoric acid (1.5 mmol/ L at 0.6 mL/min) was used as an eluent. The water content was measured by Karl Fischer titration (Metrohm 831 KF coulometer).

The elemental analysis of the anion exchange resin was carried out using an X-ray Photoelectron Spectrometer (Thermo Fisher Scientific K_{α} model). A monochromatic Al K_{α} X-ray source was used with a spot size of 400 µm at a pressure of 10⁻² Pa (10⁻⁷ mbar). A constant pass energy of 200 eV for the survey mode and 50 eV for the high-resolution region was used. The flood gun was turned on during the measurement in order to compensate the potential charging of the surface. The peak position was adjusted based on the internal standard C Is peak at 284.8 eV, with an accuracy of ± 0.05 eV. Avantage processing software was used to analyze all spectra, and the peak fitting was done on the basis of a mixed Lorentian-Gaussian function.

Thermogravimetric analysis measurements were performed with a TA Instruments thermo gravimetric analyzer from RT to 150 °C. The heating rate was 10 °C*min⁻¹ and the purge gas used was air.

### Results and Discussion

### Characterization of the anion exchange resin used for the recovery of acetate

In an example of the present method an acetate anion is recovered from a fermentation broth (at pH>pKa) with an anion exchange resin, for example Dowex Marathon MSA in Cl form, because at the pH (7.7) of the broth the salt needs to be split and the acetate is recovered. The loading of acetate to the resin was 0.043 g acetate/g of wet resin. The surface composition of the resin loaded with acetate was measured with XPS and compared with the chloride and bicarbonate form of the resin (Table 1).

**Table 1. X-ray photoelectron spectroscopy analysis of Dowex Marathon MSA resin***

| **Resin Counter-ion** | **Surface atomic composition (%)** | | | |
|---|---|---|---|---|
| | **C** | **O** | **N** | **Cl** |
| **Chloride** | 82.62 ± 0.26 | 5.40 ± 0.21 | 6.25 ± 0.26 | 5.57 ± 0.02 |
| **Acetate** | 77.31 ± 0.76 | 18.45 ± 1.42 | 2.66 ± 2.14 | Not present |
| **Bicarbonate** | 79.33 ± 0.78 | 12.88 ± 1.59 | 4.80 ± 0.52 | Not present |

| | | | | |
|---|---|---|---|---|
| * Relative constitution as C,O, N, Cl. | | | | |

Table 1 presents a surface atomic composition of the dried resin in the chloride, acetate and bicarbonate form, respectively. Note that there is no chloride detectable after loading the resin with acetate or bicarbonate using the column elution technique, which is in agreement with the chloride and acetate UV measurements in the outlet of the column. Furthermore, oxygen is present (5.40-18.45%) in all the samples. The increase of oxygen in the acetate and bicarbonate is in accordance with the inclusion of oxygen by the acetate and bicarbonate molecules. However, it was not expected to have oxygen present in the chloride form of the resin. To corroborate that there is water adsorbed to the resin, the water content of the resin before and after drying was measured with TGA. The amount of water on the resin is 68% before drying and 6.8-8% after drying. These values are used to quantify the amount of water introduce to the system by the resin. This is found important for the further desorption and esterification steps.
It is found that once the target carboxylate is bound to the resin, the desorption of the carboxylate from the anion exchange resin is limited by the strong binding energy of the electrostatic interactions between the carboxylate and the quaternary ammonium group of the resin. To solve this problem, in the next section a new approach for the desorption of the carboxylate using CO₂-expanded alcohols is explored.

### Desorption of acetate with CO₂-expanded methanol

An innovative step in the present method is the desorption of e.g. acetate from the anion exchange resin. The acetate anion is desorbed from the resin using e.g. CO₂-expanded methanol. Table 2 shows the effect of CO₂ pressure on the desorption of acetate. At low CO₂ pressures (2-10 *10⁵ Pa) a high recovery yield (mol acetic acid/mol acetateᵢₙ) ranging from 0.55-0.79 is observed. In contrast, at an equilibrium CO₂ pressure of 10*10⁵ Pa in water a desorption of 0.38±0.05 mol acetic acid/acetateᵢₙ was found (data not shown).

**Table 2. Desorption of acetate from an anion exchange resin with CO₂-expanded methanol at 20-22 °C with a resin loading of 3.5 g dry resin/ g methanol.**

| **Experiment** | **Equilibrium CO₂ pressure (bar)** | **Final water (mmol/g solvent)** | **Desorption (mol acetic acid/mol acetateᵢₙ)** |
|---|---|---|---|
| **1** | 2.12 | 0.35±0.04 | 0.55±0.09 |
| **2** | 5 | 0.37±0.07 | 0.72±0.02 |
| **3** | 10 | 0.38±0.05 | 0.79±0.04 |

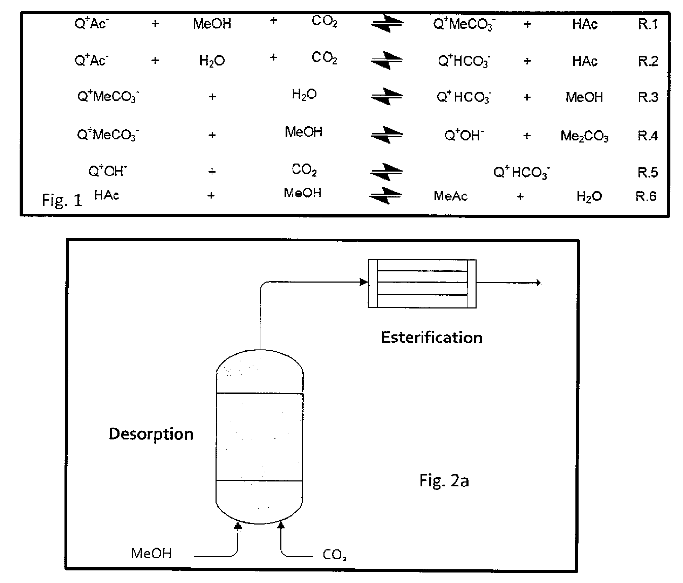

With this desorption, the anion exchange resin is regenerated to the bicarbonate form (Figure 1, R.2), and the acetate is protonated. The higher solubility of carbon dioxide in methanol enhances the desorption of acetate from the anion exchange resin. This allows the utilization of a relatively low CO₂ pressure (<2*10⁶ Pa) to protonate the carboxylate anion. On the contrary, in water with a similar low CO₂ pressure the acetate protonation is found to be driven by CaCO₃ precipitation. Downsides of using water are the co-extraction of the solvent and the limitation of using merely higher molecular alcohols (four to eight carbon chain) to further recover the acid. In contrast, in the present method also low molecular weight alcohols such as methanol can be used, which alcohols can be used further in an esterification reaction.

### Integration of esterification with CO₂-expanded methanol desorption

The esterification of acetic acid with methanol per se is a well-studied topic. It has been reported that CO₂ can improve the molar yield of esterification reactions from 0.64 to 0.72-0.80. However, reaction kinetics are not improved, and a time to reach equilibrium is long (>20 h). The reaction rate of the esterification reaction is often (too) slow and then a catalytic agent may be used. The catalytic agents that may be used are H₂SO₄, strong cation exchange resins comprising sulfonic groups, HCl, enzymes, and zeolites. Some other carboxylic acids, on the other hand, are strong enough acids that can catalyze their own esterification reaction. A type of catalyst, reaction temperature, CO₂ pressure used, and stability of the catalyst and anion exchange resin may influence a reactor configuration. Different reactor configuration considered are: a consecutive-2 compartment set-up; a simultaneous-1 compartment set-up; and a simultaneous-2 compartment set-up (Figures 2a-c). The reactor configurations are studied in detail to determine the effect of integrating the two processes.

The present experiments were performed using the simultaneous 1-compartment system and the consecutive 2-compartment systems. The experiments with the simultaneous 2-compartment system are found difficult to implement in a lab scale; results are expected to be similar to the 1-compartment system at the same operating temperature for both compartments. The main results are shown in Table 3.

**Table 3. Effect of the catalyst on the esterification of acetic acid with methanol at 60 °C for 4 h at different process configuration.**

| **Experiment** | **Process configuration** | **Solution** | **CO₂ pressure (bar)** | **Catalyst^{a,b}** | **Final water (mmol/g solvent)** | **Yield (mol methyl acetate/mol acetateᵢₙ)** |
|---|---|---|---|---|---|---|
| 1 | Only esterification | 0.4 %w/w HAc in MeOH | 0 | none | Not available | - |
| 2 | Only esterification | 0.4 %w/w HAc in MeOH | 0 | H₂SO₄ | Not available | 0.86±0.02 |
| 3 | Only esterification | 0.2%w/w HAc in MeOH | 0 | H₂SO₄ | 0.06±0.02 | 0.76±0.02 |
| 4 | Only esterification. | 0.4 %w/w HAc in MeOH | 0 | Amberlyst | 0.27±0.02 | 0.81±0.02 |
| 5 | Only esterification | 0.2%w/w HAc in MeOH | | Amberlyst | 0.12±0.01 | 0.85±0.02 |
| 6 | Consecutive | Desorbed effluent I CO₂-expanded protonation (10 bar) | 0 | H₂SO₄ | 0.60±0.03 | 0.87±0.02 |
| 7 | Simultaneous | 3.3 %w/w Dowex MSA Acetate form in MeOH | 5 | none | 0.40±0.06 | - |
| 8 | Simultaneous | 3.3 %w/w Dowex MSA Acetate form in MeOH | 5 | Amberlyst | 0.39±0.07 | 0.58±0.07 |

| | | | | | | |
|---|---|---|---|---|---|---|
| a. 0.08 % w/w H₂SO₄ b. 1.4 %w/w dry Amberlyst 15 | | | | | | |

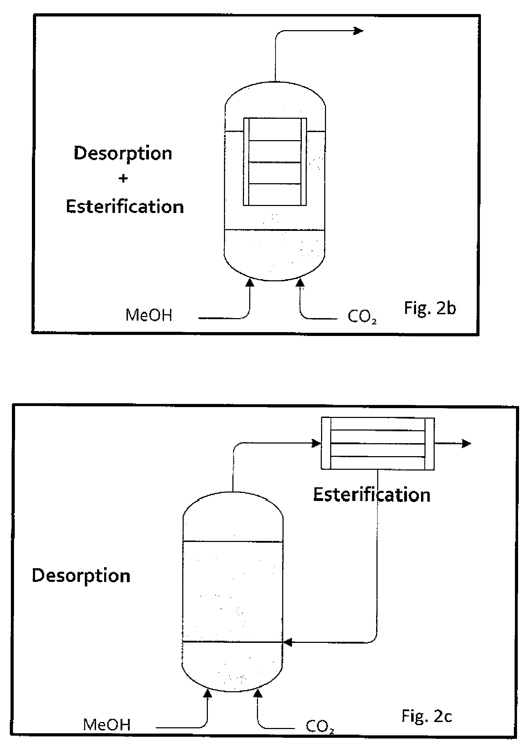

It can be observed that the maximum yield achieved is 0.85, which is similar to reported values in literature using different catalyst systems. The simultaneous desorption and esterification process configuration provided a yield of 0.58±0.07 mol methyl acetate per mole of acetateᵢₙ. This value is comparable to the consecutive step in which the desorption step yield is 0.79±0.03 mol of acetic acid per mole of acetateᵢₙ, and the further esterification reaction is 0.85±0.02 mol methyl acetate per mole of acetic acidᵢₙ. Envisaged advantages and disadvantages vary for the process integration options shown in Figure 2. For example, the 2-compartments options can potentially operate at optimum conditions for both desorption and esterification. A major advantage of the simultaneous options (1- and 2-compartments) is an equilibrium shift of the desorption step to the product side by consumption of acetic acid during esterification. This allows utilization of lower CO₂ pressures. On the other hand, a heterogeneous acid catalyst might be desired, because otherwise the acid catalysts' anion (e.g. sulfate) can exchange in the anion exchange resin in the desorption compartment. The main disadvantage of the simultaneous 1-compartment option is that the two resins used therein will have to be separated after a batch has been fully desorbed.
Water may be introduced to the reaction with the utilization of resins (anion and cation exchange) in each of the systems. The introduction of water might reduce the ester production. In order to increase the yield, a further reactive distillation step to remove the water constantly is required. As water may have a strong effect on the amount of ester produced in the esterification reaction, the effect of water concentration on the esterification was studied. Water has been removed from the resin by washing it with methanol and drying it in an oven for 60 °C. This might be an expensive step for an industrial process because of the chemical utilization and waste production. For this reason, the effect of a higher water concentration during the desorption and esterification of acetate with CO₂-expanded methanol is studied in the next section.

### Effect of water on the desorption and esterification of acetate with CO₂-expanded methanol

Water is a product from the esterification between e.g. acetic acid and methanol. Based on the equilibrium reactions, the higher the initial water concentration the lower the yield of methyl acetate. In previous experiments, the resin was washed with anhydrous methanol and dried at 60 °C in an oven. It can be advantageous for an industrial process if the resin does not have to be completely dried and the excess water can be removed only by filtration. A comparison between the yields obtained with a dry (MeOH dried) and wet resin (filtered) are presented in Table 4. The wet resin was obtained after washing the resin a couple of times with deionized water and filter the water out with a vacuum pump (-10 mbar). The water concentrations were measured with TGA (as above) and were 68-71% and 6.8-8%, respectively.

**Table 4. Effect of water on desorption and esterification of acetic acid with methanol at 60 °C for 4 h.**

| **Experiment** | **Process step** | **Resin pretreatment** | **CO₂ pressure (bar)** | **Temperature (°C)** | **Catalyst^{a,b}** | **Final water (mmol/g solvent)** | **Total desorption yield ((mol MeAc+mol HAc)/mol acetateᵢₙ)** | **Yield (mol methyl acetate/mol acetateᵢₙ)** |
|---|---|---|---|---|---|---|---|---|
| 1 | Desorption | Dry resin | 10 | 20 | none | 0.42±0.03 | 0.79±0.04 | - |
| 2 | Desorption | Wet resin | 10 | 20 | none | 1.90±0.40 | 0.50±0.01 | - |
| 3 | Esterification | Dry resin | 0 | 60 | H₂SO₄ | 0.60±0.03 | - | 0.87±0.02 |
| 4 | Esterification | Wet resin | 0 | 60 | H₂SO₄ | 1.95±0.14 | - | 0.97±0.01 |
| 5 | Desorption and Esterification | Dry resin | 5 | 60 | Amberlyst | 0.39±0.07 | 0.66±0.07 | 0.58±0.07 |
| 6 | Desorption and Esterification | Wet resin | 5 | 60 | Amberlyst | 3.42±0.5 | 0.57±0.01 | 0.37±0.02 |

| | | | | | | | | |
|---|---|---|---|---|---|---|---|---|
| a. 0.1 % w/w H₂SO₄ b. 1.4 %w/w dry Amberlyst 15 | | | | | | | | |

The desorption of acetate from the anion exchange resin is affected by the water present, and a decrease on the desorption to 0.50 mol acetic acid/ mol acetateᵢₙ is found. The reason might be the lower solubility of CO₂ in water in comparison with methanol (as discussed above). For the esterification of acetic acid with methanol a water content of 1.95±0.14 does not seem to affect the equilibrium at the lower initial acetic acid concentration used (∼1.5 mg acetic acid/ g solution). Since the concentration of acetic acid in this treatment is lower than the others, the excess methanol improves the yield until almost full conversion of methyl acetate. Interestingly, for the simultaneous desorption and esterification the total desorption is not largely affected by the water content (yield between 0.57-0.65). In the case of the esterification for the simultaneous system, the yield is reduced from 0.58±0.07 to 0.37±0.02. The extra water present in the reaction medium coming from the two different ion exchange resins affects the equilibrium and reduces the methyl acetate formation. From the TGA results, the concentration of water introduced to the system by the anion exchange resin is higher in the case of the wet resin (3.3 mmol water/ g solvent) than with the dry resin (0.16 mmol water/ g solvent). In general terms, the yield might be improved by the removal of water from the system, but also by using a higher excess of methanol to push the equilibrium to the products.
To improve the yield of produced methyl acetate, a higher excess of methanol is used in further experiments to push the equilibrium in the direction of the products. Moreover, other carboxylates (e.g. succinate and lactate) are used to prove that the technology can be applied in several applications.

### Desorption and esterification with other carboxylates: lactate and succinate

The yield of the desorption and esterification of e.g. acetate can be improved using a higher excess of methanol to push the equilibrium further. The amount of resin was decreased from 3.4 to 1.7 %w/w dry resin Dowex MSA/ methanol to increase the methyl carboxylate yields. Table 5 shows that at this condition the yield of methyl acetate formation increased to completion (1.03±0.07). The same ratio of resin and methanol was used for the desorption and esterification of succinate and lactate. These two carboxylates are attractive platform chemicals because of their chemical functionality and valuable derivatives.

**Table 5. Desorption and esterification of acetate, succinate and lactate with CO₂-expanded methanol at 60 °C and 5*10⁵ Pa (5 bar) CO₂ (a,b).**

| **Carboxylate** | **Time (h)** | **Loading of carboxylate on resin (mmol carboxylate/ g dry resin)** | **Final water (mmol/g solvent)** | **Final acid (mmol/ g solvent)** | **Yield monoester (mol methyl carboxylate/mol carboxylateᵢₙ)** | **Yield diester (mol dimethyl carboxylate/mol carboxylateᵢₙ)** |
|---|---|---|---|---|---|---|
| Acetate | 4 | 2.2 | 0.31±0.12 | 0.0034±0.0005 | 1.03 ±0.07 | - |
| Succinate | 4 | 1.8 | 0.20 | 0.0013 | 0.02 | <0.04 |
| Succinate | 20 | 1.8 | 0.32±0.04 | 0.0005 ±0.0001 | 0.017±0.003 | 0.18±0.03 |
| Lactate | 4 | 2.0 | 0.16±0.05 | 0.004±0.001 | 0.70 ±0.02 | - |

| | | | | | | |
|---|---|---|---|---|---|---|
| a. 1.7 %w/w dry resin Dowex Marathon MSA / methanol b. 1.4 %w/w dry Amberlyst 15 | | | | | | |

Table 5 shows that the desorption and esterification of succinate with CO₂-expanded methanol is possible with a modest yield of 0.18 mol dimethyl succinate/mol succinateᵢₙ at 60 °C and 5*10⁵ Pa (5 bar) CO₂. From the residual amount of succinic acid (0.0006 mmol/g) and methyl succinate (yield of 0.01 mol monoester/mol carboxylateᵢₙ) in the methanol solution, it seems that most of the dicarboxylate anion is still bounded to the resin. Such may indicate that a relative low CO₂ pressure (5 bar) is not enough to protonate the two carboxylates groups of succinate and push it out of the resin. Moreover, an increase in the yield for the esterification for 20 h suggests that a better catalyst (temperature) system for succinic acid has to be developed to achieve a high yield at shorter process times; also for esterification of succinic acid with ethanol a major difference in diethyl succinate formation might be achieved with different cation exchange resins as catalyst. However it is shown that the present method is applicable also for dicarboxylic acids separation and esterification (as well as for tricarboxylic acids), but a further optimization of the process conditions (temperature, catalyst, excess alcohol) might be needed to improve the feasibility of the process.

Some of the other carboxylates that are considered are: propionic acid, 3-hydroxypropionic acid, levulinic acid, itaconic acid, lactic acid, and 2,5-furandicarboxylic acid (FDCA). Moreover, the technology can be used with other alcohols; ethyl carboxylates are produced from the esterification with ethanol, and are widely used in industry. Specifically ethyl acetate and ethyl lactate are compounds that have several industrial applications. In the next section the formation of ethyl acetate with CO₂-expanded ethanol is shown.

### Other CO₂-expanded alcohols: ethanol as example

Above it is shown that CO₂-expanded methanol can be used for the desorption and esterification of carboxylates from a strong anion exchange resin. A main advantage for this technology is the high solubility of CO₂ in methanol. CO₂ has a high solubility in other alcohols such as ethanol. The produced ethyl carboxylates compounds are industrially interesting products.

**Table 6. Desorption and esterification of acetate with CO₂-expanded ethanol at 5*10⁵ Pa (5 bar) CO₂ for 4 h.**

| **Process Step** | **Temperature (°C)** | **Catalyst"** | **Dry Dowex MSA-acetate in ethanol (% w/w)** | **Final water (mmol/g solvent)** | **Total desorption yield ((mol EtAc + HAc)/ mol acetateᵢₙ)** | **Yield (mol ethyl acetate/mol acetateᵢₙ)** |
|---|---|---|---|---|---|---|
| Desorption | 20 | None | 3.5 | 0.16±0.01 | 0.50±0.03 | - |
| Desorption and esterification | 78 | Amberlyst | 3.5 | 0.27±0.04 | 0.71±0.02 | 0.55±0.02 |
| Desorption and esterification | 78 | Amberlyst | 1.7 | 0.30±0.15 | 0.87±0.05 | 0.67±0.04 |

| | | | | | | |
|---|---|---|---|---|---|---|
| a. 1.4 %w/w dry Amberlyst 15 | | | | | | |

Table 6 shows the results for the desorption and esterification of acetate with CO₂-expanded ethanol at 5*10⁵ Pa (5 bar). The desorption yield at 20 °C is 0.50±0.03, which is lower than the amount achieved with methanol (0.72±0.02). This might be because of steric effect of the bulkier ethyl group in comparison with the methyl group during ethyl carbonate and methyl carbonate formation. Similar results were reported for the transesterification of ethylene carbonate with different alcohols, in which the yield decreases with an increase of the carbon atoms of the alcohol. This seems to indicate that a relatively higher pressure is required for CO₂-expanded ethanol to achieve higher desorption yields. As in the methanol case, the desorption yield increases when a simultaneous esterification occurs. The desorption increases to 0.71±0.02 mol (HAc + MeAc)/mol acetateᵢₙ with an esterification yield of 0.52±0.02 with an excess of ethanol of 3.5%w/w acetateᵢₙ/ethanol. A further excess of ethanol (1.7%w/w acetateᵢₙ/ethanol) increases the esterification yield to 0.63±0.01, which is close to expected.
These experiments confirmed that the technology is applicable for several alcohols, for example butanol, isoamyl alcohol, and geraniol can be used to produce esters such as butyl butyrate, isoamyl acetate and geranyl acetate, which are important in the fragrance and flavor industry.

### FIGURES

The invention although described in detailed explanatory context may be best understood in conjunction with the accompanying figures.
Fig. 1 shows exemplary reactions R1-R6.
Figs. 2a-c show reactor set-ups.

### DETAILED DESCRIPTION OF THE FIGURES

The figures have been detailed throughout the description.

## Claims

1. Method of recovery of carboxylates comprising the steps of
providing the carboxylate in an aqueous solution, performing an anion exchange sorption step using a anion exchange resin or ionic liquid capturing the carboxylate, and
desorbing the carboxylate and protonating the carboxylate with a solution comprising a CO₂ expanded alcohol, and simultaneously regenerating the resin or ionic liquid to a bicarbonate form thereof.

2. Method according to claim 1, wherein the carboxylate is obtained from biotransformation, such as a fermentation process, from a microbial process, from hydrolysis of biomass, from a chemical conversion, or from waste water, wherein the aqueous solution preferably comprises a broth.

3. Method according to any of the preceding claims, wherein the carboxylates are a combination of different carboxylates.

4. Method according to any of the preceding claims, wherein the carboxylates (and carboxylic acids thereof) are C₂-C₂₀ carboxylates, optionally comprising at least one further carboxylate group, such as 1-2 further carboxylate groups, and/or a further comprising at least one alcohol group, such as 1-2 alcohol groups, and/or optionally being cyclic, such as comprising a cyclohexane or cyclopentane group, and/or comprising an aromatic moiety, such as a benzene group, such as formic acid, acetic acid, propionic acid, butyric acid, valeric acid, caproic acid, enanthic acid, caprylic acid, pelargonic acid, capric acid, undecanoic acid, lauric acid, tridecyclic acid, myristic acid, pentadecanoic acid, palmitic acid, margaric acid, stearic acid, nonadecyclic acid, arachidic acid, lactic acid, succinic acid, levulinic acid, itaconic acid, 2,5-furandicarboxylic acid (FDCA), phthalic acid, acrylic acid, adipic acid, benzoic acid, fumaric acid, 3-hydroxy butyric acid, malic acid, pimelic acid, 3-hydroxyproprionic acid, sugar acids such as uronic acids and aldonic acids, vanillic acid, ferulic acid, and alginic acid.

5. Method according to any of the preceding claims, wherein a pH of the aqueous solution is controlled by addition of a suitable amount of base selected from at least one of oxide, hydroxide, bicarbonate and carbonate, preferably bicarbonate and carbonate.

6. Method according to claim 6, wherein the base is liberated from the resin during sorption of the carboxylate, and/or wherein the pH of the aqueous solution is larger than the pKₐ of the acid of the carboxylate, preferably pH>(pKₐ +0.5).

7. Method according to any of the preceding claims, wherein the alcohol comprises at least one of a C₁-C₁₂ straight or branched, and/or optionally being cyclic, primary or secondary alcohol, such as methanol, ethanol, propanol, isopropanol, butanol, heptanol, 3-methyl-1-butanol, (trans)-3,7-Dimethyl-2,6-octadien-1-ol, and hexanol, and optionally one of water-miscible solvents selected from acetone, acetonitrile, and tetrahydrofuran.

8. Method according to any of the preceding claims, wherein a CO₂ pressure is from 0,5-200 * 10⁵ Pa (0,5-200 bar), and/or wherein the desorption is performed at a temperature of 0-100 °C, preferably 20-60 °C.

9. Method according to any of the preceding claims, wherein the protonated carboxylate is dissolved in the CO₂ expanded alcohol, and/or
wherein the protonated carboxylate is esterified under homogeneous acid or heterogeneous catalyst conditions.

10. Method according to claim 9, wherein a catalytic agent is provided, selected from H₂SO₄, HCl, a cation exchange resin comprising sulfonic groups, enzymes, and zeolites.

11. Method according to any of claims 9-10, wherein esterification is performed during a period of 0.1 -10 h, preferably 0.2-5 h, more preferably 0.5-2 h.

12. Method according to any of the preceding claims, wherein the method is carried out in a simultaneous 1-compartment system, in a consecutive 2-compartment system, or in a simultaneous 2-compartment system.

13. Method according to any of the preceding claims, wherein the carboxylic acid in the CO₂ expanded alcohol is recovered, such as by at least one of evaporation of the alcohol and CO₂, crystallization of the carboxylic acid, and anti-solvent addition.

14. Method according to any of the preceding claims, wherein prior to the anion exchange desorption step water is removed from the resin, preferably removed to a concentration of less than 1% (w/w water/resin).

15. Method according to any of the preceding claims, wherein water is removed, such as by reactive distillation.
